# EUROPEAN PATENT APPLICATION

(11) **EP 4 616 889 A1**
(43) Date of publication of application: **17.09.2025**
(21) Application number: 24305377.4
(22) Date of filing: 12.03.2024
(51) Int. Cl.: A61M 5/32

(54) **MULTI-MATERIAL SYRINGE NEEDLE SHIELD AND METHOD OF MANUFACTURING THEREOF**

(71) Applicant: Becton Dickinson France, 38800 Le Pont-de-Claix (FR)
(72) Inventor: PERROUD, Corentin, 38500 Voiron (FR); CHAGNEUX, Alexandra, 38600 Fontaine (FR); VAXELAIRE, Jérémie, 74100 Ambilly (FR); REMPFER, Simon, 38260 St Hilaire de la Côte (FR); OZTURK, Senturk, 38130 Echirolles (FR); DINC, Mirhac, 74960 Annecy (FR); DEVILLARD, Sylvain, 38000 Grenoble (FR)
(74) Representative: Germain Maureau

(57) **Abstract**

Provided herein is a needle shield for use with a syringe comprising a needle and a barrel. The needle shield includes an elastomeric skin layer configured to receive the needle therein and maintain tightness between the needle shield and the needle when the needle shield is positioned on the syringe, and a rigid core embedded within the elastomeric skin layer.

## Description

### BACKGROUND OF THE INVENTION

### Field of the Invention

The present disclosure relates generally to syringes and, more particularly, to a needle shield for a syringe, with the needle shield comprising a multi-material needle shield formable via a co-injection or bi-injection method.

### Description of Related Art

Syringes are used in a variety of environments for administering fluids, such as medications or other drugs, to a patient. Many syringes are provided as prefilled syringes, which provide the convenience of rapidly delivering the liquid therein to a patient without the need to first aspirate the medication from another container and meter its volume. The prefilled syringe will typically include a syringe barrel having a distal end and a proximal end. A needle is connected at the distal end for injecting fluid into the patient and a plunger assembly is inserted through the proximal end that is movable within the barrel to force fluid out from the syringe through the needle.

It is recognized that syringes must be handled with care before and after use due to the presence of the needle. To minimize the risk of accidental injury due to needle sticks, the syringe typically includes a needle shield that covers the sharpened tip of the needle. The needle shield is removed prior to use to expose the sharpened tip of the needle. Such a shield also serves to protect the sharpened tip of the needle, ensure product tightness, and preserve the sterility of the needle prior to use of the injection device.

Needle shields are generally formed to include a rigid component and a soft component or "jupe." The soft component is typically formed of elastic rubber (e.g., isoprene rubber, butyl rubber, latex rubber, silicone rubber or the like) that provides a secure sealing connection with the syringe, at least along a sealing line, and the rigid component provides protection against accidental needle sticks and provides the user with a grippable surface for the user to remove the needle shield from the syringe.

While existing needle shields and the soft component therein provide adequate protection to the needle, it is recognized that existing needle shield designs may present drawbacks with regard to manufacturing and assembly thereof and/or with regard to performance of the syringe.

As one example, manufacturing of a needle shield is typically performed as a three-step process. First and second steps of the manufacturing process involve the forming of the rigid component and the soft component as two separate pieces and via two separate molding processes. A third step of the manufacturing process then involves assembly of the rigid component and the soft component into a single needle shield, with the soft component being positioned within the rigid component and secured therein. Such a three-step process not only leads to an increased manufacturing and assembly time and cost, but may also lead to issues regarding improper assembly of the rigid component and the soft component and/or issues regarding the rigid component and the soft component becoming disconnected from one another during storage or use due to an improper joining securing of the components.

As another example, existing needle shields - and specifically the soft component thereof - can present issues of "coring" of the soft component into the needle. That is, it is recognized that when the needle shield is applied onto the syringe, the needle is pricked into the soft component of the needle shield to ensure a jupe-needle interface tightness and sealing. However, pricking of the needle into the soft component may cause a coring to occur, where a portion of the rubber (or other elastomeric material) is cut from the soft component and lodged within the lumen of the needle. This core of elastomeric material may thus clog the needle and negatively impact the performing of a subsequent injection using the syringe. Additionally, in some instances, the coring of the soft component by the needle can damage the beveled tip of the needle and/or alter a silicon layer applied onto the beveled tip - with any of these issues potentially impacting penetration of the needle into the patient, such as by increasing a required penetration force or increasing discomfort to a patient.

Accordingly, a need exists in the art for a needle shield that provides a simplified manufacturing and assembly process. A need also exists in the art for a needle shield that reduces the potential for coring to occur between the needle and the soft component of the needle shield.

### SUMMARY OF THE INVENTION

Provided herein is a needle shield for use with a syringe comprising a needle and a barrel. The needle shield includes an elastomeric skin layer configured to receive the needle therein and maintain tightness between the needle shield and the needle when the needle shield is positioned on the syringe and a rigid core embedded within the elastomeric skin layer.

In accordance with some aspects of the disclosure, the elastomeric skin layer comprises a closed distal end and an open proximal end and defines a cavity therein extending distally from the open proximal end, with the elastomeric skin layer forming an outer surface of the needle shield.

In accordance with some aspects of the disclosure, the elastomeric skin layer comprises a cylindrical body defining the cavity therein, and wherein the core comprises a tubular member embedded within the cylindrical body.

In accordance with some aspects of the disclosure, the cylindrical body of the elastomeric skin layer includes a depression formed on a distally-facing external surface thereof, at the closed distal end, and wherein the tubular member includes a hook-shaped end member at a distal end thereof, with the hook-shaped end member extending proximally and radially inward from the distal end of the tubular member and matching a profile of the depression.

In accordance with some aspects of the disclosure, the elastomeric skin layer comprises a tapered elongated body defining the cavity therein and tapering from the open proximal end to the closed distal end, and wherein the core comprises a tapered tubular member embedded within the cylindrical body that matches a profile of the tapered elastomeric skin layer.

In accordance with some aspects of the disclosure, the elastomeric skin layer is formed of a thermoplastic elastomer.

In accordance with some aspects of the disclosure, the rigid core is formed of a rigid polymeric material.

Also provided herein is a method of manufacturing a needle shield for use with a syringe comprising a needle and a barrel. The method comprises performing a co-injection molding process, using a single mold, to form the needle shield, with the needle shield including an elastomeric skin layer configured to receive the needle therein and maintain tightness between the needle shield and the needle when the needle shield is positioned on the syringe and a rigid core embedded within the elastomeric skin layer.

In accordance with some aspects of the disclosure, wherein the co-injection molding process comprises a laminar injection molding.

In accordance with some aspects of the disclosure, the co-injection molding process comprises injecting an initial shot of elastomeric material into the mold to form an initial portion of the elastomeric skin layer, injecting a shot of polymeric material into the mold to form the rigid core, with the rigid core embedded within the initial portion of the elastomeric skin layer, and injecting a final shot of elastomeric material into the mold to form the elastomeric skin layer.

In accordance with some aspects of the disclosure, the final shot of elastomeric material forms the closed distal end of the elastomeric skin layer.

In accordance with some aspects of the disclosure, the elastomeric material is provided from a first material source and injected into the mold via a first material feed path; and wherein the polymeric material is provided from a second material source and injected into the mold via a second material feed path.

Also provided herein is a needle shield for use with a syringe comprising a needle and a barrel. The needle shield includes a rigid outer casing forming at least a portion of an outer surface of the needle shield and a bi-material inner casing positioned within the rigid outer casing and formed of an elastomeric material. The bi-material inner casing further includes a needle receiving portion configured to receive the needle therein when the needle shield is positioned on the syringe and a mating portion configured to engage the barrel to couple the needle shield to the syringe, wherein the needle receiving portion has a first hardness and the mating portion has a second hardness, the first hardness being lower than the second hardness.

In accordance with some aspects of the disclosure, the first hardness of the needle receiving portion comprises a Shore A hardness of 45 or less, and wherein the second hardness of the mating portion comprises a Shore A hardness of 60 or more.

In accordance with some aspects of the disclosure, each of the needle receiving portion and the mating portion is formed of an elastic rubber.

In accordance with some aspects of the disclosure, each of the needle receiving portion and the mating portion is formed of a thermoplastic elastomer.

In accordance with some aspects of the disclosure, the bi-material inner casing comprises a closed distal end and an open proximal end and defines a cavity therein, wherein the mating portion comprises the open proximal end, and wherein the needle receiving portion comprises at least part of the closed distal end.

In accordance with some aspects of the disclosure, the needle receiving portion comprises a cylindrical member surrounded circumferentially by the mating portion, with the needle receiving portion extending longitudinally from the cavity to a distal surface of the bi-material inner casing.

In accordance with some aspects of the disclosure, the needle receiving portion comprises a cylindrical member surrounded circumferentially by the mating portion, with the needle receiving portion extending longitudinally from the cavity to a location that is inset proximally from a distal surface of the bi-material inner casing.

In accordance with some aspects of the disclosure, the needle receiving portion comprises an entirety of the closed distal end
Also provided herein is a method of forming a needle shield for use with a syringe comprising a needle and a barrel with the needle shield including a rigid outer casing forming at least a portion of an outer surface of the needle shield and a bi-material inner casing positioned within the rigid outer casing and formed of an elastomeric material, and with the bi-material inner casing further including a needle receiving portion configured to receive the needle therein when the needle shield is positioned on the syringe and a mating portion configured to engage the barrel to couple the needle shield to the syringe, wherein the needle receiving portion has a first hardness and the mating portion has a second hardness, the first hardness being lower than the first hardness. The method includes performing a first molding process to form the rigid outer casing, performing a second molding process to form the bi-material inner casing, and securing the bi-material inner casing within the rigid outer casing.

In accordance with some aspects of the disclosure, performing the second molding process comprises performing a bi-inj ection molding, with the needle receiving portion comprising a first thermoplastic elastomer having the first hardness and the mating portion comprising a second thermoplastic elastomer having the second hardness.

In accordance with some aspects of the disclosure, performing the second molding process comprises performing a first compression molding to form the needle receiving portion from a first elastic rubber having the first hardness; performing a second compression molding to form the mating portion from a second elastic rubber having the second hardness; and bonding the needle receiving portion to the mating portion, to form the bi-material inner casing.

### BRIEF DESCRIPTION OF THE DRAWINGS

FIG. 1 is a perspective view of a syringe including a needle shield, according to an embodiment of the disclosure;
FIG. 2 is an exploded view of the syringe of FIG. 1;
FIG. 3A is a perspective view of a syringe including a needle shield, according to an embodiment of the disclosure;
FIG. 3B is a cross-sectional view of the needle shield of FIG. 3A;
FIG. 4A is a perspective view of a syringe including a needle shield, according to an embodiment of the disclosure;
FIG. 4B is a cross-sectional view of the needle shield of FIG. 4A;
FIG. 5A is a perspective view of a syringe including a needle shield, according to an embodiment of the disclosure;
FIG. 5B is a cross-sectional view of the needle shield of FIG. 5A;
FIGS. 6A-6D illustrate process steps for forming a needle shield included in the syringe of FIG. 1;
FIG. 7 is a cross-sectional view of a needle shield useable with the syringe of FIG. 1, according to an embodiment of the disclosure;
FIG. 8 is a cross-sectional view of a needle shield useable with the syringe of FIG. 1, according to another embodiment of the disclosure;
FIG. 9 is a cross-sectional view of a needle shield useable with the syringe of FIG. 1, according to another embodiment of the disclosure;
FIG. 10 is a cross-sectional view of a needle shield useable with the syringe of FIG. 1, according to another embodiment of the disclosure; and
FIG. 11. illustrates a molding assembly for forming any of the needle shields of FIGS. 7-10, according to an embodiment of the disclosure.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

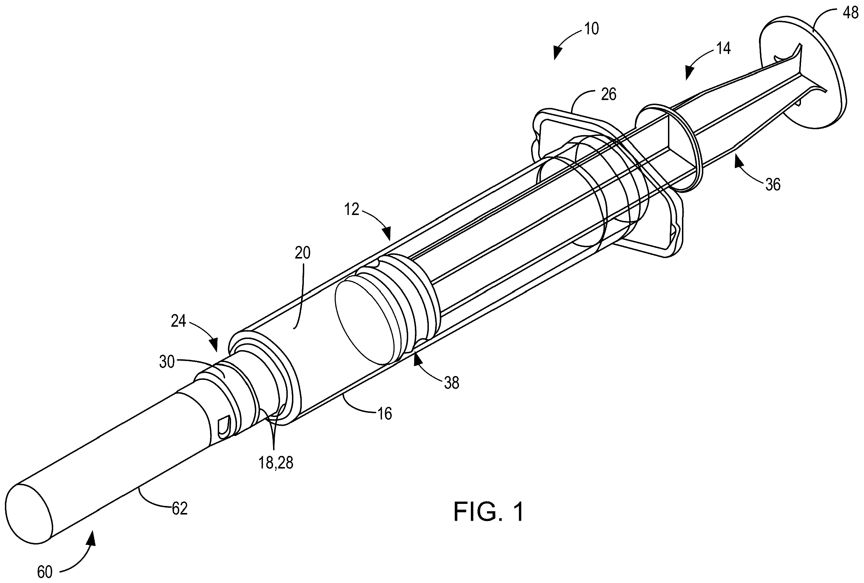

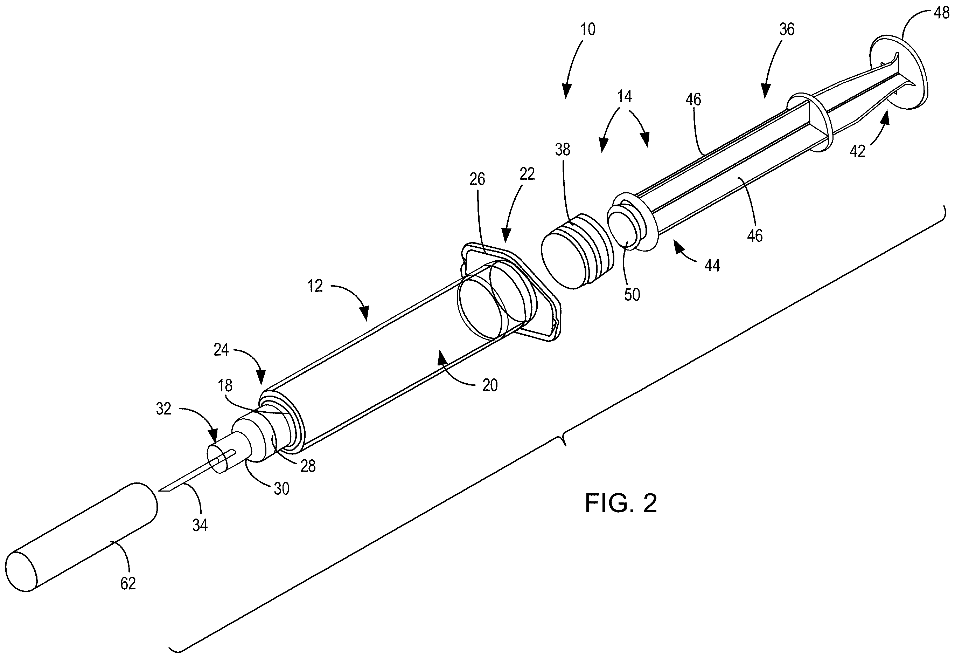

Referring to FIGS. 1 and 2, shown is a non-limiting embodiment of a medical injection device 10, such as a syringe for example, with which aspects or embodiments of the disclosure may be implemented. While the medical injection device is shown and described hereafter as a syringe 10, it is recognized that other medical injection devices may incorporate aspects of the disclosure, as described in detail here below.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical outer wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion 30 extending out distally from shoulder 28. The hub portion 30 is formed as a partially hollow member that defines a channel 32 therethrough in fluid communication with the chamber 20. A needle 34 is attached to the hub portion 30 within channel 32, with a proximal end of the needle 34 positioned within the channel 32 and a distal end of the needle 34 extending out from the hub portion 30. The proximal end of the needle 342 may be inserted into the channel 32 of hub portion 30 and may be fixed therein with an adhesive (e.g., glue), by thermal welding, or the like. The needle 34 defines a hollow lumen therein that is in fluid communication with the chamber 20 of syringe barrel 12, such that fluid may be dispensed from the syringe 10.

The plunger assembly 14 of syringe 10 is formed of an elongate plunger rod 36 (more generally "plunger 36," as used hereafter) and a plunger head or stopper 38. The plunger 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger 36 with respect to the syringe barrel 12. In some embodiments, a flanged extension member 50 (e.g., disc-shaped flange) is positioned at the plunger distal end 44 that is configured to mate with the stopper 38. In other embodiments, the plunger distal end 44 may include a female receptacle formed therein that is configured to receive and connect to a protrusion (e.g., pin) extending out proximally from the stopper, with the protrusion and receptacle engaging via threading, for example.

The stopper 38 of plunger assembly 14 is positioned at the plunger distal end 44 so as to be movable along with the plunger 36 within the chamber 20 of syringe barrel 12. The stopper 38 may be made from a material that is different from the material of the plunger 36 and that is capable of forming a tight seal with the syringe barrel 12 as it is advanced therethrough. In some embodiments, the stopper 38 includes a receptacle (not shown) formed therein that is sized and configured to receive the flanged extension member 50 of plunger 36, with the flanged extension member 50 coupling with the receptacle via a press fit connection, for example, to secure the stopper 38 to the plunger 36, although it can be appreciated that the stopper 38 and plunger distal end 44 can be secured by other techniques known in the art.

As shown in FIGS. 1 and 2, a needle shield 60 of syringe assembly 12 is coupled to the syringe barrel 12 to protect the needle 34. According to aspects of the disclosure, the needle shield 60 may be composed of both a rigid material that provides structure to the needle shield 60 and an elastomeric material (i.e., a deformable or compliant material) that surrounds the needle 34 when the needle shield 60 is coupled to the syringe barrel 12. In various embodiments, the rigid material may be polypropylene (PP), polyethylene (PE), polyethylene terephthalate (PET), polystyrene (PS) or polycarbonate (PC), as non-limiting examples, while the elastomeric material may be a thermoplastic elastomer (TPE), such as TPS - SEBS (Styrene-Ethylene-Butylene-Styrene) materials or TPV Santoprene 101-55, or any others elastomeric material proccessable by co-injection, as non-limiting examples.

According to aspects of the disclosure, the needle shield 60 may be manufactured via a co-injection molding process, so as to allow for forming of the needle shield 60 as a one-shot molded component, with two distinct materials - i.e., the rigid material and elastomeric material. That is, a single mold is utilized to produce a composite needle shield 60 formed of the rigid material and elastomeric material. A "skin" layer of the needle shield 60 may be formed of an elastomeric material, while a core of the needle shield 60 may be formed of a rigid material - with the rigid core entirely/completely embedded within the elastomeric skin layer 62. With the needle shield 60 formed as a single co-injected molded component, manufacturing thereof may be simplified, as only a single step process is needed, versus a three-step process of individually/separately molding a rigid shield portion and an elastomeric shield portion, and then assembling the separate rigid and elastomeric shield portions. Forming of the needle shield as a single co-inj ected molded component also eliminates potential issues regarding improper assembly of separate rigid and elastomeric shield portions and/or spontaneous disconnection of the rigid and elastomeric shield portions that may occur over time.

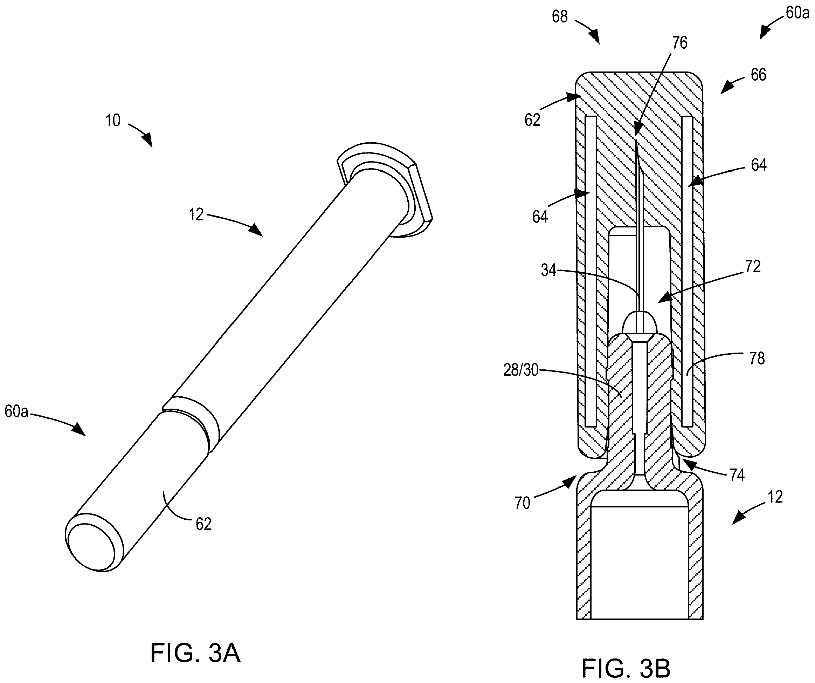

FIGS. 3A and 3B, FIGS. 4A and 4B and FIGS. 5A and 5B illustrate various embodiments of co-injected needle shields 60 that may be formed, according to aspects of the disclosure. In each of the embodiments, the needle shields 60 are formed as a single co-injecting component, with a rigid core entirely/completely embedded within an elastomeric skin layer 62. The co-injected needle shields 60 provide an improved feel (i.e., softness) due to the elastomeric skin layer 62 forming an outer surface of the needle shield, as compared to prior art needle shields where a rigid material forms the outer surface. The co-injected needle shields 60 also function to maintain a tightness function at interfaces between the needle tip and the elastomeric skin layer 62 and between the syringe barrel hub and the elastomeric skin layer 62, so as to ensure protect the sharpened tip of the needle and preserve its sterility and also provide a secure sealing connection with the syringe.

Referring to FIGS. 3A and 3B, a needle shield 60a is shown according to one embodiment of the disclosure. The needle shield generally includes a skin layer 62 formed of an elastomeric material and a core 64 formed of a rigid material. As previously described, the rigid material may be polypropylene (PP), polyethylene (PE), polyethylene terephthalate (PET), polystyrene (PS) or polycarbonate (PC), as non-limiting examples, while the elastomeric material may be a thermoplastic elastomer (TPE).

As shown in FIG. 3B, the skin layer 62 may be generally structured as a cylindrical body 66 having a closed distal end 68 and an open proximal end 70. A hollow cavity 72 is formed in the cylindrical body 66 at the open proximal end 70 thereof, with an opening 74 in the cylindrical body 66 providing access into the cavity 72. The cavity 72 extends distally into the cylindrical body 66 a portion of the length of the cylindrical body 66. The cavity 72 is sized and positioned to receive at least a portion of the needle 34 therein, to provide protection to the needle 34 once the needle shield 60 is coupled to the syringe barrel 12. In some embodiments, the cavity 72 is configured such that, when the needle shield 60 is secured on syringe barrel 12, the needle 34 extends through a length of the cavity 72, such that a needle tip 76 protrudes through the cavity 72 to reach the elastomeric material of closed distal end 68 that is positioned distally from cavity 72 - i.e., the needle tip 76 punctures and extends into the elastomeric material - such that the needle tip 76 is sealed in the elastomeric material of the skin layer 62. With the needle tip 76 sealed in the elastomeric material of the skin layer 62, leakage of fluid out from the needle 34 may be prevented.

As shown in FIG. 3B, the core 64 is constructed as a tubular member 78 that is embedded within the skin layer 62. The core 64 may extend a majority of a length of the overall needle shield 60a, from a location adjacent the closed distal end 68 to a location adjacent the open proximal end 70. The core 64 functions to add stiffness to the overall needle shield 60a - preventing an excessive amount of bending, flexing or overall deformation of the skin layer 62.

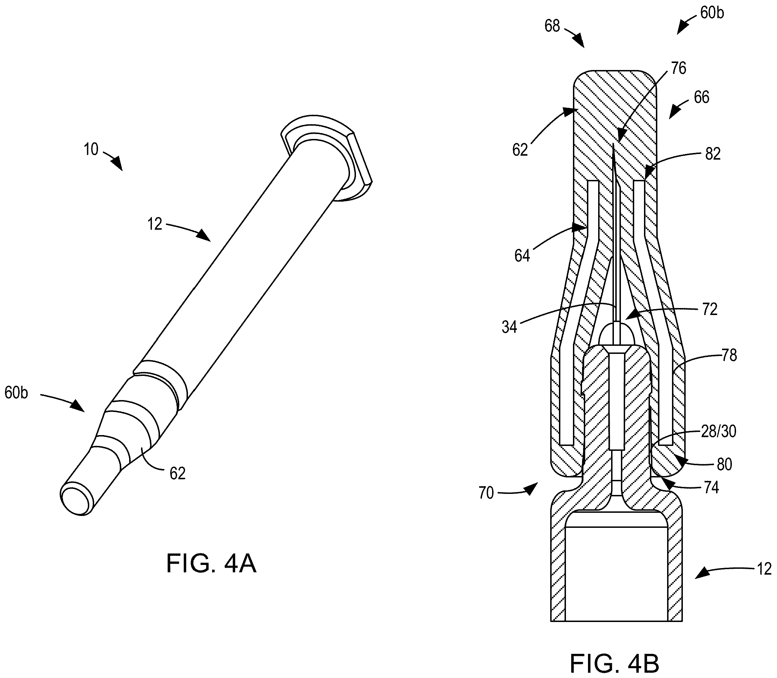

Referring to FIGS. 4A and 4B, a needle shield 60b is shown according to another embodiment of the disclosure. The needle shield 60b includes a skin layer 62 formed of an elastomeric material and a core 64 formed of a rigid material - with the skin layer 62 and core 64 formed of any of a number of suitable materials previously described. The needle shield 60b has a generally similar construction to the needle shield 60a of FIGS. 3A and 3B, with the skin layer 62 structured as an elongated body 66 having a closed distal end 68 and an open proximal end 70, and with a hollow cavity 72 formed in the elongated body at the open proximal end 70 that extends distally into the elongated body 66 a portion of the length thereof. However, in needle shield 60b, the elongated body 66 is formed as a tapered body having a diameter that reduces proximally-to-distally - i.e., is tapered from the open proximal end 70 to the closed distal end 68.

As shown in FIG. 4B, with the skin layer 62 constructed as a tapered body, the core 64 is also constructed as a tapered member that generally matches a profile of the skin layer 62. The core 64 may again have a tube-like structure 78 (i.e., with an open proximal end 80 and open distal end 82), but is constructed so as to taper from the proximal end 80 to the distal end 82. The core 64 may extend a majority of a length of the overall needle shield 60b, from a location adjacent the closed distal end 68 of skin layer 62 to a location adjacent the open proximal end 70 of skin layer 62. The core 64 functions to add stiffness to the overall needle shield 60b - preventing an excessive amount of bending, flexing or overall deformation of the skin layer 62.

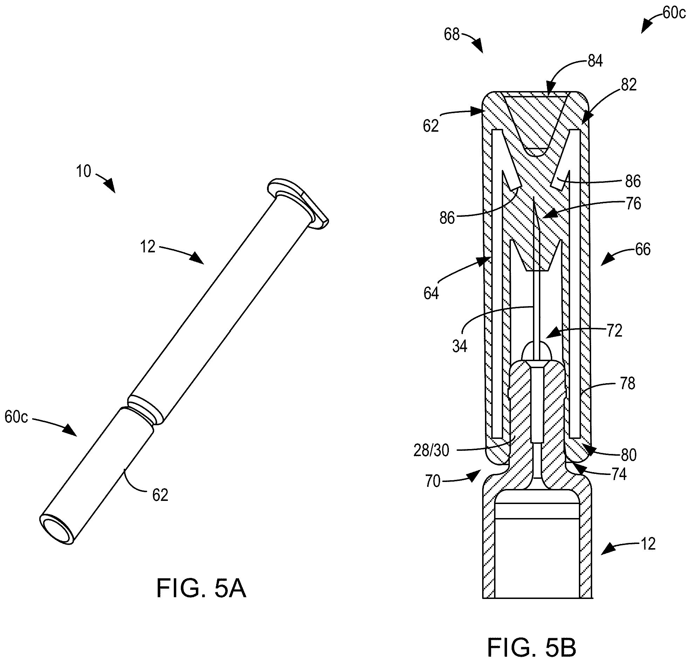

Referring to FIGS. 5A and 5B, a needle shield 60c is shown according to another embodiment of the disclosure. Again, the needle shield 60c includes a skin layer 62 formed of an elastomeric material and a core 64 formed of a rigid material - with the skin layer 62 and core 64 formed of any of a number of suitable materials previously described. The needle shield 60c has a generally similar construction to the needle shield 60a of FIGS. 3A and 3B, with the skin layer 62 structured as a cylindrical body 66 having a closed distal end 68 and an open proximal end 70, and with a hollow cavity 72 formed in the cylindrical body 66 at the open proximal end 70 that extends distally into the cylindrical body 66 a portion of the length thereof. However, in needle shield 60c, the cylindrical body 66 is formed so that the closed distal end 68 includes an indent or depression 84 formed on a distally-facing external surface thereof, with the indent/depression 84 reducing the amount of elastomeric material required to form the skin layer 62. According to embodiments, the indent/depression 84 may be formed as a bowl-shaped depression or a conical depression, as non-limiting examples.

As shown in FIG. 5B, with the skin layer 62 constructed as a cylindrical body 66 with a distal end depression 84 formed therein, the core 64 may be constructed to include features that coincide with the depression. That is, the core 64 may be constructed as a generally tubular member 78 that is embedded within the skin layer 62, but that includes a hook-shaped end member 86 at the distal end thereof. The hook-shaped end member 86 may extend proximally and radially inward from a distal end 82 of the tubular member 78, with a profile of the hook-shaped end member 86 generally matching the profile of the depression 84 formed in the skin layer 62. The core 64 may extend a majority of a length of the overall needle shield 60c, from a location adjacent the closed distal end 68 to a location adjacent the open proximal end 70. The core 64 functions to add stiffness to the overall needle shield 60c - preventing an excessive amount of bending, flexing or overall deformation of the skin layer 62.

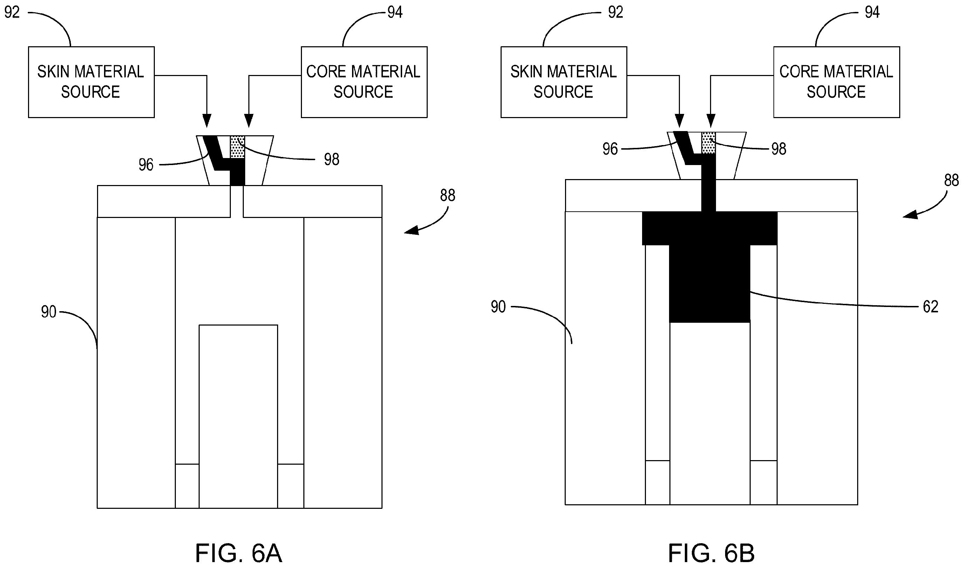

Referring now to FIGS. 6A-6D, and with continued reference to FIGS. 3-5, a co-injection molding process for forming a needle shield 60 is described in further detail. While the needle shield 60 illustrated in FIGS. 6A-6D coincides with the needle shield 60a of FIGS. 3A and 3B, it is recognized that the co-injection molding process described here below could also be used to form the needle shield 60b of FIGS. 4A and 4B or the needle shield 60c of FIGS. 5A and 5B.

As shown in FIGS. 6A-6D, a system 88 for manufacturing the needle shield 60 generally includes a mold 90, a first material source 92 that provides the elastomeric skin material, and a second material source 94 that provides the rigid core material. Both material sources 92, 94 are arranged to selectively introduce material into the mold 90 to form the needle shield 60. As explained in further detail below, the system 88 provides a laminar or "fountain" flow of materials that ensure that the material at the front of the co-injection molding shots forms the skin layer 62 of the needle shield 60, while the material behind that fills the core 64.

As shown in FIG. 6A a co-injection molding process begins with the mold 90 in an empty state.

The co-injection molding process continues as shown in FIG. 6B, where skin material is injected from the first material source 92 and into a portion of the mold 90 via a first material feed path 96. As previously described, the skin material is an elastomeric material that may be an injectable thermoplastic elastomer (TPE). The skin material may be injected under acceptable pressure and temperature conditions, with a mold temperature being between 10 and 50° C, and preferably between 20 and 40° C, and a material processing melt temperature being between 150 and 260° C, and preferably between 190 and 230° C, as a non-limiting example, with it recognized that other mold temperatures and material processing melt temperatures may be used, depending on the materials used to manufacture the needle shield 60.

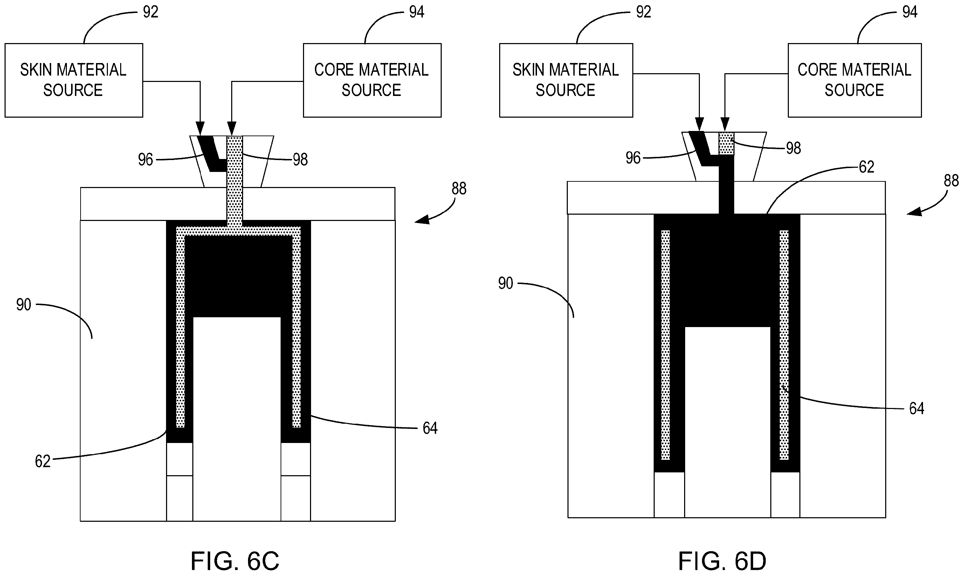

After an initial injection/shot of skin material is injected into a portion of the mold 90, the co-injection molding process continues as shown in FIG. 6C, where core material is injected from the second material source 94 and into a portion of the mold 90 via a second material feed path 98. As previously described, the core material is a rigid material that may be polypropylene (PP), polyethylene (PE), polyethylene terephthalate (PET), polystyrene (PS) or polycarbonate (PC), as non-limiting examples. The core material is injected as part of a laminar or "fountain" flow of the skin and core materials that ensures that the elastomeric material at the front of the co-injection molding shots forms the skin layer 62 of the needle shield 60, while the rigid material behind that fills the core 64 and is embedded in the skin layer 62. The core material may be injected under acceptable pressure and temperature conditions, with a mold temperature being between 20 and 40° C, and a material processing melt temperature being between 175 and 230° C or between 220 and 260° C, as a non-limiting example.

With the rigid core material injected into the mold 90, the co-injection molding process continues as shown in FIG. 6D, where an additional/final shot of skin material is injected from the first material source 92 into the mold 90. The final shot of skin material may function to fill-in and form the closed end of the skin layer 62, so as to provide a deformable pricking area in which the sharpened tip of the needle 34 may be retained. Accordingly, a co-injected needle shield 60 is thus formed that includes an elastomeric skin layer 62 forming an outer surface of the needle shield 60, so as to provide an improved feel (i.e., softness) for the shield, along with a rigid inner core 64 that adds structure and durability to the needle shield 60.

According to some aspects of the disclosure, it is recognized that it may be desirable to form the soft component or "jupe" of a needle shield from a combination of different elastomeric materials. That is, in some instances with existing needle shield designs, it is possible that the jupe can present issues of "coring", where a portion of the elastomeric material is cut from the jupe and lodged within the lumen of the needle when the needle is pricked into the jupe to ensure a jupe-needle interface tightness and sealing. The presence or extent of such coring may depend of the particular hardness/softness of the jupe material, with it recognized that a stiffer jupe material may be desired for an interface between the needle shield and syringe barrel hub (to promote securing/sealing therebetween), but that a jupe with too high a stiffness may increase the chances for coring to occur. Accordingly, it would be desirable to provide a jupe for a needle shield that exhibits multiple hardness/softness characteristics, by way of a bi-material jupe being provided.

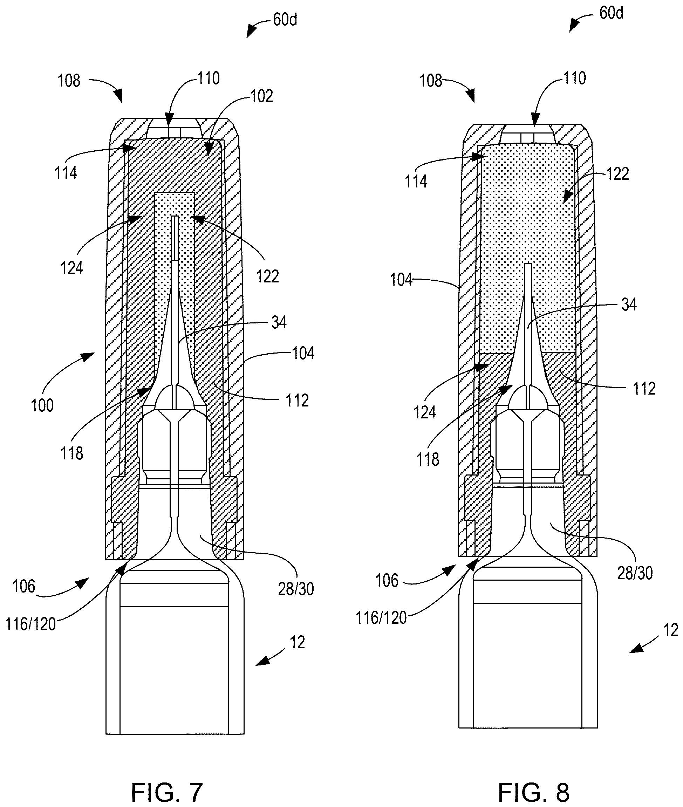

FIGS. 7-10 illustrate various embodiments of needle shields 60d that include a bi-material jupe therein. In each of the embodiments, the needle shields 60d are composed of a rigid outer casing 100 that provides structure to the needle shield 60d and a compliant inner casing or jupe 102 that surrounds the needle 34 (when needle shield 60d is coupled to syringe barrel 12).

As shown in FIGS. 7-10, the outer casing 100 is constructed as a tubular member 104 that includes an open proximal end 106 and an open distal end 108. The open distal end 108 may include an opening 110 therein having a smaller diameter than a diameter of the compliant jupe 102, such that the jupe 102 may be better retained in the rigid outer casing 100.

The jupe 102 includes an elongated body 112 having a closed distal end 114 and an open proximal end 116. A hollow cavity 118 is formed in the elongated body 112 at the open proximal end 116 thereof, with an opening 120 in the elongated body 112 providing access into the cavity 118. The cavity 118 extends distally into the elongated body 112 a portion of the length of the elongated body 112. The cavity 118 may have an inner diameter that is smaller than an outer diameter of the hub 30 (FIGS. 1 and 2), such that the hub 30 is prevented from sliding into cavity 118 when the needle shield 60d is secured to syringe barrel 12. The cavity 118 is sized and positioned to receive at least a portion of the needle 34 therein, to provide protection to the needle 34 once the needle shield 60d is coupled to the syringe barrel 12. In some embodiments, the cavity 118 is configured such that, when the needle shield 60d is secured on syringe barrel 12, the needle 34 extends through a length of the cavity 118, such that a needle tip 76 protrudes through the cavity 118 to reach the compliant material of closed distal end 114 that is positioned distally from cavity 118 - i.e., the needle tip 76 punctures and extends into the compliant material - such that the needle tip 76 is sealed in the compliant material of the jupe 102. With the needle tip 76 sealed in the compliant material of the jupe 102, leakage of fluid out from the needle 34 may be prevented.

As described above, the jupe 102 is formed of two distinct elastomeric materials. A first portion 122 of the jupe 102 may thus be formed of a first elastomeric material, while a second portion 124 of the jupe 102 may be formed of a second elastomeric material. According to embodiments, the first portion 122 of the jupe 102 may form part of (FIGS. 7, 9 and 10) or all of (FIG. 8) the closed distal end 114 of the jupe 102. In all embodiments, the first portion 122 may be characterized as a "needle receiving portion" that encompass at least that portion of the closed end into which needle tip 76 punctures and extends into the jupe 102. The second portion 124 may then form a remainder of the jupe 102, including the open proximal end 116 that may be secured to the hub 30 of syringe barrel 12 when the needle shield is secured on the syringe and/or including or otherwise presenting at least a portion of the an outer surface of the jupe 102 that may be mated with and secured to the outer rigid casing 100, such that the second portion 124 may be characterized as a "mating portion."

In the embodiment of FIG. 7, the first portion 122 of the jupe 102 is formed as a cylindrical member that is surrounded circumferentially by the second portion 124, such that the first portion 122 forms only a part of the closed distal end 114. The first portion 122 forms a portion of the wall of the cavity 118 and extends distally from the cavity 118 to a location that is inset proximally from a distal surface of the closed distal end 114.

In the embodiment of FIG. 8, the first portion 122 of the jupe 102 is formed as a cylindrical member that is positioned distally from the second portion 124. The first portion 122 extends distally from the cavity 118 and forms an entirety of the closed distal end 114 of jupe 102.

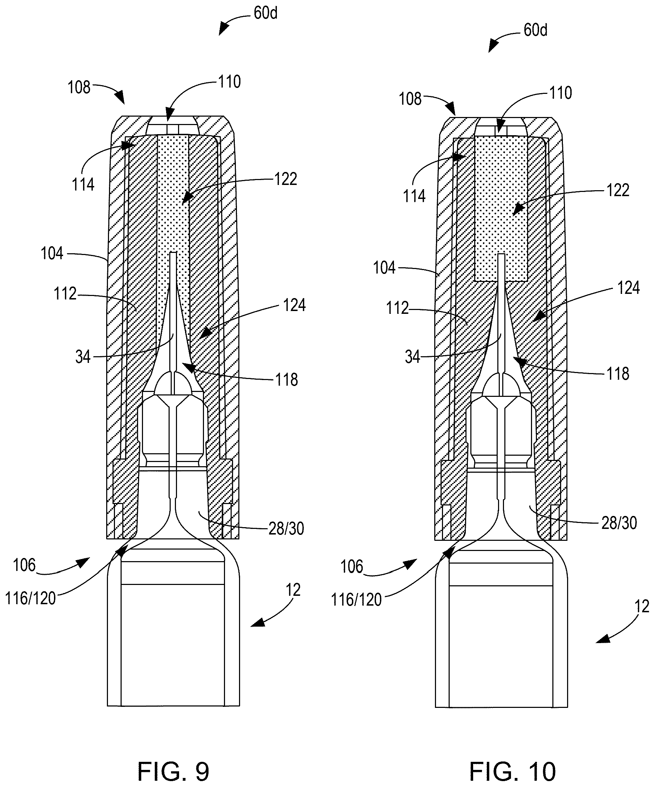

In the embodiment of FIG. 9, the first portion 122 of the jupe 102 is formed as a cylindrical member that is surrounded circumferentially by the second portion 124, such that the first portion 122 forms only a part of the closed distal end 114. The first portion 122 forms a portion of the wall of the cavity 118 and extends distally from the cavity 118 to the distal surface of the closed distal end 114.

In the embodiment of FIG. 10, the first portion 122 of the jupe 102 is formed as a cylindrical member that is surrounded circumferentially by the second portion 124, such that the first portion 122 forms only a part of the closed distal end 114. The first portion 122 extends distally from a distal end of the cavity 118 and to the distal surface of the closed distal end 114.

According to aspects of the disclosure, the first material that forms the first portion 122 of jupe 102 has a shore hardness that is lower than the shore hardness of the second material that forms the second portion 124 of jupe 102 - i.e., the first material is softer than the second material. The lower shore hardness of the first material - in the region of jupe 102 into which needle tip 76 punctures and extends - reduces the potential for (1) coring or chipping of the material to occur when the needle is inserted into the first material, and/or (2) damaging of the needle bevel when the needle is inserted into the first material, as the softer first material is more able to deform and "give" when the needle 34 is inserted therein. The higher shore hardness of the second material - in regions of the jupe 102 other than where the needle tip 76 punctures and extends - increases the stability of the jupe 102 and retention thereof within rigid outer casing, and helps in forming a seal with the hub 30 of the syringe barrel 12.

According to some embodiments, the first material may be Mediprene resine grade (TPS) 500434M-02 or Santoprene 8281-XXMED (TPV) (where XX = 35 / 45), as non-limiting examples, while the second material may be Medipren resine grade (TPS) 500XX4M-02 (where XX = 63 / 68 / 73) or Santoprene 8281-XXMED (TPV) (where XX = 65 / 75 / 90), as non-limiting examples. In some non-limiting embodiments, the first material preferably has a Shore A hardness of 45 of less, while the second material preferably has a Shore A hardness of 60 or more. With these hardness values, the jupe 102 may provide the functionality as described above.

According to aspects of the disclosure, the bi-material jupe 102 may be formed via a number of different manufacturing processes.

In one embodiment, the bi-material jupe 102 may be formed via a bi-injection molding (i.e., overmolding) process - where two different thermoplastic elastomer (TPE) materials are injected into a rotating mold. In such a bi-injection process, two separate TPE materials of differing hardness/softness are injected into a single mold over two separate injection cycles - with the second/harder material first being injected, followed by injection of the first/softer material. As indicated above, a first material having a lower Shore A hardness (e.g., 45 or less) is used to form the first portion 122 of the jupe 102, while a second material having a higher Shore A hardness (e.g., 60 or more) is used to form the second portion 124 of the jupe 102.

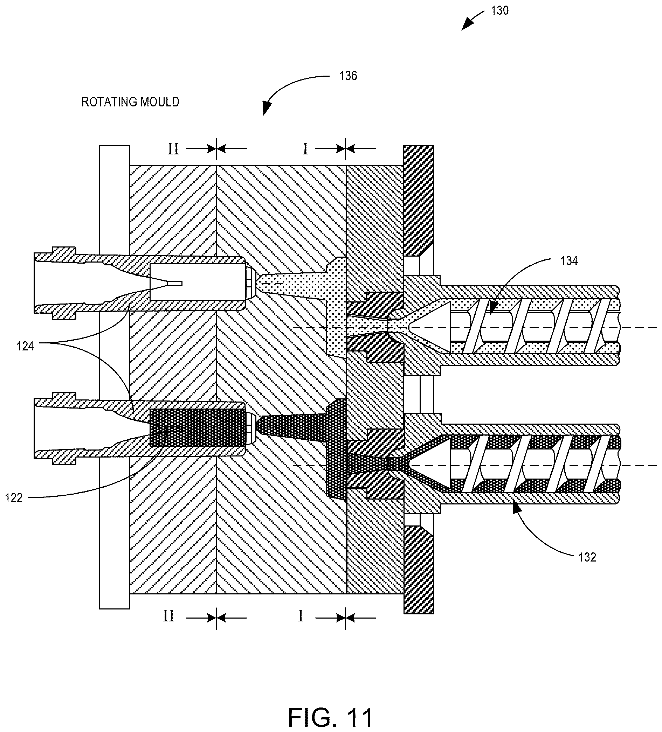

Referring to FIG. 11, a molding assembly 130 that may be used for forming a needle shield 60d with a bi-material jupe 102 via a bi-injection molding process is described in further detail. While the molding assembly 130 is illustrated for forming the bi-material jupe 102 of FIG. 10, it is recognized that the molding assembly 130 could be configured to form any of the jupes 102 illustrated in FIGS. 7-10. The molding assembly 130 includes a first injection unit 132 for injecting a softer first material (that forms first portion 122 of jupe 102) and a second injection unit 134 for injecting a harder second material (that forms second portion 124 of jupe 102). The injection units 130, 132 inject material into a rotating mold 136 that enables a bi-injection or overmolding process to be performed. In formation of the bi-material jupe 102, the second injection unit 134 is controlled to initially inject the harder second material into the mold 136, so as to form the second portion 124 of jupe 102. After injection of the harder second material, the mold 136 is rotated, so as to reposition the formed second portion 124 and allow for an overmolding of the first material thereon. After rotation, the first injection unit 132 is controlled to inject the softer first material into the mold 136, so as to form the first portion 122 of jupe 102. The completed bi-material jupe 102 is thus formed via operation of the molding assembly 130.

In another embodiment, the bi-material jupe 102 may be formed via a compression molding and bonding process - where the first portion 122 and the second portion 124 are separately formed via two distinct compression molding processes using different materials. That is, a first material, such as an elastic rubber (e.g., isoprene rubber, butyl rubber, latex rubber, silicone rubber or the like), is used to form the first portion 122, with the first portion 122 formed via a compression molding process, while a second material, such as an elastic rubber (e.g., isoprene rubber, butyl rubber, latex rubber, silicone rubber or the like), is used to form the second portion 124, with the second portion 124 formed via another compression molding process. The separately formed first portion 122 and second portion 124 may then be bonded together, such as via an adhesive, to form the bi-material jupe 102.

Beneficially, embodiments of the disclosure provide a needle shield that may be formed via a single co-injection molding process, with the needle shield providing an improved feel (i.e., softness) due to the elastomeric skin layer 62 forming an outer surface of the needle shield, while ensuring protection and sterility of the sharpened tip of the needle. Additional embodiments of the disclosure provide a needle shield having a bi-material jupe, with a first portion of the jupe (that receives the needle tip therein) formed of a first material having a Shore A hardness that is lower than that of a second material that is used to form a second portion of the jupe (that mates with the rigid outer cap and/or syringe barrel), with the first material of the jupe reducing the potential for coring of the jupe by the needle and/or damaging of the needle bevel.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A needle shield for use with a syringe comprising a needle and a barrel, the needle shield comprising:
an elastomeric skin layer configured to receive the needle therein and maintain tightness between the needle shield and the needle when the needle shield is positioned on the syringe; and
a rigid core embedded within the elastomeric skin layer.

2. The needle shield of claim 1, wherein the elastomeric skin layer comprises a closed distal end and an open proximal end and defines a cavity therein extending distally from the open proximal end, with the elastomeric skin layer forming an outer surface of the needle shield.

3. The needle shield of claim 2, wherein the elastomeric skin layer comprises a cylindrical body defining the cavity therein, and wherein the rigid core comprises a tubular member embedded within the cylindrical body.

4. The needle shield of claim 3, wherein the cylindrical body of the elastomeric skin layer includes a depression formed on a distally-facing external surface thereof, at the closed distal end, and wherein the tubular member includes a hook-shaped end member at a distal end thereof, with the hook-shaped end member extending proximally and radially inward from the distal end of the tubular member and matching a profile of the depression.

5. The needle shield of claim 4, wherein the elastomeric skin layer comprises a tapered elongated body defining the cavity therein and tapering from the open proximal end to the closed distal end, and wherein the rigid core comprises a tapered tubular member embedded within the cylindrical body that matches a profile of the tapered elastomeric skin layer.

6. The needle shield of claim 1, wherein the elastomeric skin layer is formed of a thermoplastic elastomer.

7. The needle shield of claim 1, wherein the rigid core is formed of a rigid polymeric material.

8. A method of manufacturing the needle shield of claims 1-9, the method comprising performing a co-injection molding process, using a single mold, to form the needle shield.

9. The method of claim 8, wherein the co-injection molding process comprises a laminar injection molding.

10. The method of claim 8, wherein the co-injection molding process comprises:
injecting an initial shot of elastomeric material into the mold to form an initial portion of the elastomeric skin layer;
injecting a shot of polymeric material into the mold to form the rigid core, with the rigid core embedded within the initial portion of the elastomeric skin layer; and
injecting a final shot of elastomeric material into the mold to form the elastomeric skin layer.

11. The method of claim 10, wherein the final shot of elastomeric material forms the closed distal end of the elastomeric skin layer.

12. The method of claim 10, wherein the elastomeric material is provided from a first material source and injected into the mold via a first material feed path; and wherein the polymeric material is provided from a second material source and injected into the mold via a second material feed path.

13. A needle shield for use with a syringe comprising a needle and a barrel, the needle shield comprising:
a rigid outer casing forming at least a portion of an outer surface of the needle shield; and
a bi-material inner casing positioned within the rigid outer casing and formed of an elastomeric material, the bi-material inner casing comprising:
a needle receiving portion configured to receive the needle therein when the needle shield is positioned on the syringe; and
a mating portion configured to engage the barrel to couple the needle shield to the syringe;
wherein the needle receiving portion has a first hardness and the mating portion has a second hardness, the first hardness being lower than the second hardness.

14. The needle shield of claim 13, wherein the first hardness of the needle receiving portion comprises a Shore A hardness of 45 or less, and wherein the second hardness of the mating portion comprises a Shore A hardness of 60 or more.

15. The needle shield of claim 13, wherein each of the needle receiving portion and the mating portion is formed of an elastic rubber.

16. The needle shield of claim 13, wherein each of the needle receiving portion and the mating portion is formed of a thermoplastic elastomer.

17. The needle shield of claim 13, wherein the bi-material inner casing comprises a closed distal end and an open proximal end and defines a cavity therein, wherein the mating portion comprises the open proximal end, and wherein the needle receiving portion comprises at least part of the closed distal end.

18. The needle shield of claim 17, wherein the needle receiving portion comprises a cylindrical member surrounded circumferentially by the mating portion, with the needle receiving portion extending longitudinally from the cavity to a distal surface of the bi-material inner casing.

19. The needle shield of claim 17, wherein the needle receiving portion comprises a cylindrical member surrounded circumferentially by the mating portion, with the needle receiving portion extending longitudinally from the cavity to a location that is inset proximally from a distal surface of the bi-material inner casing.

20. The needle shield of claim 17, wherein the needle receiving portion comprises an entirety of the closed distal end.

21. A method of forming the needle shield of claims 13-20, the method comprising:
performing a first molding process to form the rigid outer casing;
performing a second molding process to form the bi-material inner casing; and
securing the bi-material inner casing within the rigid outer casing.

22. The method of claim 21, wherein performing the second molding process comprises performing a bi-injection molding, with the needle receiving portion comprising a first thermoplastic elastomer having the first hardness and the mating portion comprising a second thermoplastic elastomer having the second hardness.

23. The method of claim 21, wherein performing the second molding process comprises:
performing a first compression molding to form the needle receiving portion from a first elastic rubber having the first hardness;
performing a second compression molding to form the mating portion from a second elastic rubber having the second hardness; and
bonding the needle receiving portion to the mating portion, to form the bi-material inner casing.
